# EUROPEAN PATENT APPLICATION

(11) **EP 4 684 733 A1**
(43) Date of publication of application: **28.01.2026**
(21) Application number: 24779180.9
(22) Date of filing: 05.03.2024
(51) Int. Cl.: A61B 6/00, A61B 6/12, A61B 6/46, A61B 8/12

(54) **IMAGE PROCESSING DEVICE, IMAGE PROCESSING SYSTEM, IMAGE DISPLAY METHOD, AND IMAGE PROCESSING PROGRAM**

(30) Priority: 31.03.2023 JP 2023057180
(71) Applicant: Terumo Kabushiki Kaisha, Tokyo 151-0072 (JP)
(72) Inventor: SHIMIZU Katsuhiko, Ashigarakami-gun, Kanagawa 259-0151 (JP); YOSHIZAWA Shunsuke, Ashigarakami-gun, Kanagawa 259-0151 (JP)
(74) Representative: Prüfer & Partner mbB Patentanwälte · Rechtsanwälte
(86) International application number: PCT/JP2024/008371
(87) International publication number: WO 2024/203044

(57) **Abstract**

An image processing device receives a calibration operation of adjusting a viewpoint when a three-dimensional image obtained by imaging a three-dimensional structure of a biological tissue based on the basis of tomographic information obtained by a sensor that moves in a lumen of the biological tissue is displayed on a display in accordance with a radiation direction of an X-ray, adjusts relative sizes and positions of an acquired X-ray image and the three-dimensional image on the basis of ratio information and position information each time an X-ray image obtained by fluoroscopically viewing the biological tissue is acquired, and causes the display to display the acquired X-ray image and the three-dimensional image so as to overlap each other as viewed from a viewpoint based on the viewpoint information.

## Description

### Technical Field

The present disclosure relates to an image processing device, an image processing system, an image display method, and an image processing program.

### Background Art

Patent Literature 1 discloses a method for specifying a ventral direction of a patient in a three-dimensional image when a three-dimensional image of a biological tissue is generated and displayed on the basis of a two-dimensional ultrasound image acquired by a transducer of an ultrasound catheter.

### Citation List

### Patent Literature

Patent Literature 1: WO 2022/045182 A1

### Summary of Invention

### Technical Problem

At the time of catheter surgery such as a stent graft indwelling procedure, in order to reduce X-ray exposure or assist catheter operation, it is conceivable that a three-dimensional CT image captured before surgery is merged with a DSA image generated in real time and displayed. "CT" is an abbreviation for computed tomography. "DSA" is an abbreviation for digital subtraction angiography. The DSA image is obtained by subtracting a mask image obtained by fluoroscopically viewing a blood vessel before administration of a contrast medium from a contrast image obtained by fluoroscopically viewing the blood vessel after administration of the contrast medium. By merging the three-dimensional CT image into the DSA image, a detailed shape of the blood vessel and a real-time state can be simultaneously confirmed. However, when a hard guidewire or catheter enters the blood vessel, the blood vessel may be stretched, creating a large gap between the three-dimensional CT image and the DSA image. X-ray exposure during imaging of the three-dimensional CT image may also be a problem.

An object of the present disclosure is to reduce a gap between an X-ray image and a three-dimensional image when the X-ray image obtained by fluoroscopically viewing a biological tissue using X-rays and the three-dimensional image obtained by imaging a three-dimensional structure of the biological tissue are superimposed and displayed.

### Solution to Problem

Some aspects of the present disclosure are shown below.

[1] An image processing device including:
   a control unit that generates a three-dimensional image obtained by imaging a three-dimensional structure of a biological tissue based on tomographic information obtained by a sensor that moves in a lumen of the biological tissue, sequentially acquires X-ray images obtained by fluoroscopically viewing the biological tissue using an X-ray, receives a calibration operation of adjusting a viewpoint when the three-dimensional image is displayed on a display in accordance with a radiation direction of the X-ray, and acquires ratio information regarding relative sizes of each of X-ray images and the three-dimensional image displayed on the display, and position information regarding relative positions of each of the X-ray images and the three-dimensional image displayed on the display; and
   a storage unit that stores viewpoint information regarding a viewpoint adjusted by the calibration operation, and the ratio information and the position information acquired by the control unit, in which
   each time an X-ray image is acquired, the control unit adjusts relative sizes and positions of the acquired X-ray image and the three-dimensional image on the basis of the ratio information and the position information stored in the storage unit, and then causes the display to display the acquired X-ray image and the three-dimensional image so as to overlap each other as viewed from a viewpoint based on the viewpoint information stored in the storage unit.
[2] The image processing device according to [1], in which
   the control unit acquires at least one X-ray image, stores two or more positions in a first direction in the at least one X-ray image in the storage unit as first mark positions, and stores two or more positions in a direction corresponding to the first direction in the three-dimensional image in the storage unit as second mark positions, and
   the first mark positions and the second mark positions stored in the storage unit are referred to when setting relative sizes and positions of each of the X-ray images and the three-dimensional image displayed on the display.
[3] The image processing device according to [2], in which the control unit receives a size setting operation of setting relative sizes of each of the X-ray images and the three-dimensional image displayed on the display in a state where the first mark positions and the second mark positions stored in the storage unit are displayed on the display together with the at least one X-ray image and the three-dimensional image, and acquires information regarding the relative sizes set by the size setting operation as the ratio information.
[4] The image processing device according to [2], in which the control unit performs size setting processing of setting relative sizes of each of the X-ray images and the three-dimensional image displayed on the display by adjusting relative sizes of each of the at least one X-ray image and the three-dimensional image so that a distance between two first positions included in the first mark positions stored in the storage unit coincides with a distance between two second positions corresponding to the two first positions included in the second mark positions stored in the storage unit, and acquires information regarding the relative sizes set by the size setting processing as the ratio information.
[5] The image processing device according to any one of [2] to [4], in which the control unit receives a position setting operation of setting relative positions of each of the X-ray images and the three-dimensional image displayed on the display in a state where the first mark positions and the second mark positions stored in the storage unit are displayed on the display together with the at least one X-ray image and the three-dimensional image, and acquires information regarding the relative positions set by the position setting operation as the position information.
[6] The image processing device according to [5], in which the control unit stores two or more positions in a second direction orthogonal to the first direction in the at least one X-ray image in the storage unit as third mark positions, stores two or more positions in a direction corresponding to the second direction in the three-dimensional image in the storage unit as fourth mark positions, and receives the position setting operation in a state where the third mark positions and the fourth mark positions stored in the storage unit are displayed on the display together with the at least one X-ray image and the three-dimensional image.
[7] The image processing device according to [5], in which the control unit receives the position setting operation in a state where a two-dimensional image obtained by imaging a cross-sectional structure of the biological tissue based on the tomographic information is superimposed on a predetermined position of the three-dimensional image and displayed on the display.
[8] The image processing device according to any one of [2] to [4], in which the control unit performs position setting processing of setting relative positions of each of the X-ray images and the three-dimensional image displayed on the display by adjusting relative positions of the at least one X-ray image and the three-dimensional image so that a position of the sensor in the at least one X-ray image overlaps with a position corresponding to the position of the sensor in the three-dimensional image after relative sizes of the at least one X-ray image and the three-dimensional image is adjusted so that a distance between two first positions included in the first mark positions stored in the storage unit coincides with a distance between two second positions corresponding to the two first positions included in the second mark positions stored in the storage unit, and acquires information regarding the relative positions set by the position setting processing as the position information.
[9] The image processing device according to any one of [2] to [4], in which the control unit performs position setting processing of setting relative positions of each of the X-ray images and the three-dimensional image displayed on the display by adjusting relative positions of the at least one X-ray image and the three-dimensional image so that a position of a medical instrument inserted into the biological tissue separately from the sensor in the at least one X-ray image overlaps with a position corresponding to the position of the medical instrument in the three-dimensional image after relative sizes of the at least one X-ray image and the three-dimensional image is adjusted so that a distance between two first positions included in the first mark positions stored in the storage unit coincides with a distance between two second positions corresponding to the two first positions included in the second mark positions stored in the storage unit, and acquires information regarding the relative positions set by the position setting processing as the position information.
[10] The image processing device according to [9], in which the medical instrument is a guide wire.
[11] The image processing device according to any one of [2] to [10], in which the control unit receives a sensor position designation operation of designating a position of the sensor changed with movement of the sensor in the at least one X-ray image twice or more, stores designated positions in the storage unit as the first mark positions, and stores positions corresponding to the position of the sensor when the sensor position designation operation is received in the three-dimensional image in the storage unit as the second mark positions.
[12] The image processing device according to any one of [2] to [10], in which the control unit performs sensor position detection processing of detecting a position of the sensor changed with movement of the sensor in the at least one X-ray image twice or more, stores detected positions in the storage unit as the first mark positions, and stores positions corresponding to the position of the sensor when the sensor position detection processing is performed in the three-dimensional image in the storage unit as the second mark positions.
[13] The image processing device according to any one of [2] to [10], in which
   the biological tissue is branched to form side branches at two or more locations separated in a major axis direction in the lumen, and
   the control unit performs side branch position detection processing of detecting positions of the side branches in the three-dimensional image, and stores detected positions in the storage unit as the second mark positions.
[14] The image processing device according to [13], in which, when causing the display to display each of the X-ray images and the three-dimensional image so as to overlap each other as viewed from the viewpoint, the control unit further causes the display to display side branch information regarding the side branches.
[15] The image processing device according to any one of [2] to [14], in which, when causing the display to display each of the X-ray images and the three-dimensional image so as to overlap each other as viewed from the viewpoint, the control unit further causes the display to display tube diameter information regarding a tube diameter of the lumen at the second mark positions.
[16] The image processing device according to any one of [1] to [15], in which the control unit causes the display to display each of the X-ray images and the three-dimensional image so as to overlap each other as viewed from the viewpoint by arranging each of the X-ray images on a back side of the three-dimensional image while making the three-dimensional image translucent.
[17] The image processing device according to any one of [1] to [15], in which the control unit causes the display to display each of the X-ray images and the three-dimensional image so as to overlap each other as viewed from the viewpoint by arranging each of the X-ray images in front of the three-dimensional image while making each of the X-ray images translucent.
[18] The image processing device according to any one of [1] to [17], in which, when causing the display to display each of the X-ray images and the three-dimensional image so as to overlap each other as viewed from the viewpoint, the control unit causes the display to display only a contour of the three-dimensional image.
[19] An image processing system including: the image processing device according to any one of [1] to [18]; and the display.
[20] An image display method including:
   generating a three-dimensional image obtained by imaging a three-dimensional structure of a biological tissue based on tomographic information obtained by a sensor that moves in a lumen of the biological tissue;
   sequentially acquiring X-ray images obtained by fluoroscopically viewing the biological tissue using an X-ray;
   receiving a calibration operation of adjusting a viewpoint when the three-dimensional image is displayed on a display in accordance with a radiation direction of the X-ray; and
   acquiring ratio information regarding relative sizes of each of X-ray images and the three-dimensional image displayed on the display, and position information regarding relative positions of each of the X-ray images and the three-dimensional image displayed on the display, in which
   each time an X-ray image is acquired, relative sizes and positions of the acquired X-ray image and the three-dimensional image are adjusted on the basis of the ratio information and the position information, and then the display is caused to display the acquired X-ray image and the three-dimensional image so as to overlap each other as viewed from the viewpoint adjusted by the calibration operation.
[21] An image processing program for causing a computer to execute an operation including:
   generating a three-dimensional image obtained by imaging a three-dimensional structure of a biological tissue based on tomographic information obtained by a sensor that moves in a lumen of the biological tissue;
   sequentially acquiring X-ray images obtained by fluoroscopically viewing the biological tissue using an X-ray;
   receiving a calibration operation of adjusting a viewpoint when the three-dimensional image is displayed on a display in accordance with a radiation direction of the X-ray; and
   acquiring ratio information regarding relative sizes of each of X-ray images and the three-dimensional image displayed on the display, and position information regarding relative positions of each of the X-ray images and the three-dimensional image displayed on the display, in which
   each time an X-ray image is acquired, relative sizes and positions of the acquired X-ray image and the three-dimensional image are adjusted on the basis of the ratio information and the position information, and then the display is caused to display the acquired X-ray image and the three-dimensional image so as to overlap each other as viewed from the viewpoint adjusted by the calibration operation.

### Advantageous Effects of Invention

According to the present disclosure, it is possible to reduce a gap between an X-ray image and a three-dimensional image when the X-ray image obtained by fluoroscopically viewing a biological tissue using X-rays and the three-dimensional image obtained by imaging a three-dimensional structure of the biological tissue are superimposed and displayed.

### Brief Description of Drawings

Fig. 1 is a block diagram illustrating a configuration of an image processing system according to an embodiment of the present disclosure.
Fig. 2 is a view illustrating an example of processing performed by the image processing system according to the embodiment of the present disclosure before an X-ray image and a three-dimensional image are superimposed.
Fig. 3 is a view illustrating an example of processing performed when the image processing system according to the embodiment of the present disclosure superimposes an X-ray image and a three-dimensional image.
Fig. 4 is a block diagram illustrating a configuration of an image processing device according to the embodiment of the present disclosure.
Fig. 5 is a flowchart illustrating an operation of the image processing device according to the embodiment of the present disclosure.
Fig. 6 is a flowchart illustrating a specific procedure of processing of step S2 in Fig. 5.
Fig. 7 is a flowchart illustrating a specific procedure of processing of step S4 in Fig. 5.
Fig. 8 is a flowchart illustrating a modification of the specific procedure of the processing of step S4 in Fig. 5.
Fig. 9 is a flowchart illustrating another modification of the specific procedure of the processing of step S4 in Fig. 5.
Fig. 10 is a view illustrating another example of the processing performed by the image processing system according to the embodiment of the present disclosure before the X-ray image and the three-dimensional image are superimposed.
Fig. 11 is a flowchart illustrating a specific procedure of processing of step S5 in Fig. 5.
Fig. 12 is a flowchart illustrating a modification of the specific procedure of the processing of step S5 in Fig. 5.
Fig. 13 is a view illustrating an example of a result of superimposing an X-ray image and a three-dimensional image by the image processing system according to the embodiment of the present disclosure.
Fig. 14 is a view illustrating another example of a result of superimposing the X-ray image and the three-dimensional image by the image processing system according to the embodiment of the present disclosure.
Fig. 15 is a view illustrating still another example of the result of superimposing the X-ray image and the three-dimensional image by the image processing system according to the embodiment of the present disclosure.

### Description of Embodiments

Hereinafter, an embodiment of the present disclosure will be described with reference to the drawings.

In the drawings, the same or corresponding parts are denoted by the same reference numerals. In the description of the present embodiment, the description of the same or corresponding parts will be omitted or simplified as appropriate.

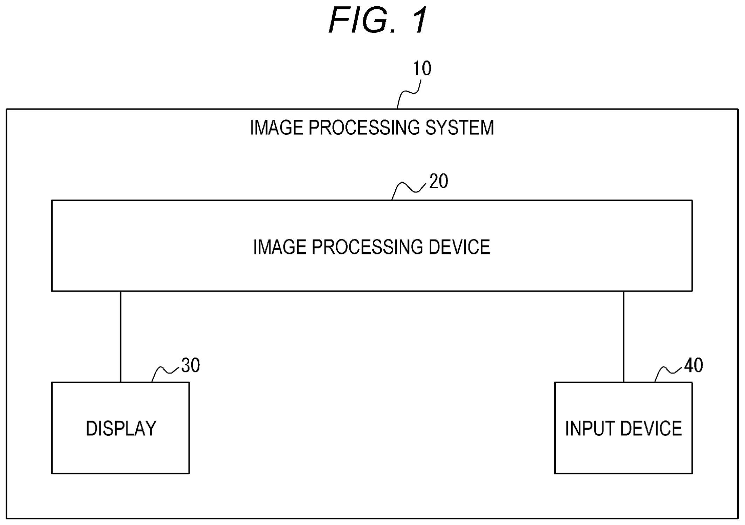

A configuration of an image processing system 10 according to the present embodiment will be described with reference to Fig. 1.

The image processing system 10 includes an image processing device 20, a display 30, and an input device 40. The image processing device 20 is connected to the display 30 and the input device 40 via a cable or a network, or wirelessly.

The image processing device 20 is, for example, a general-purpose computer such as a PC, a server computer such as a cloud server, or a dedicated computer. "PC" is an abbreviation for personal computer. The image processing device 20 may be installed in a medical facility such as a hospital, or may be installed in a facility different from the medical facility such as a data center.

The display 30 is, for example, an LCD or an organic EL display. "LCD" is an abbreviation for liquid crystal display. "EL" is an abbreviation for electro luminescent. The display 30 is installed in a medical facility and displays various types of information including images for assisting an operator such as a doctor or a clinical engineer in catheter surgery such as a stent graft indwelling procedure.

The input device 40 is, for example, a pointing device such as a mouse, a keyboard, or a touch screen disposed integrally with the display 30. The input device 40 is installed in a medical facility and is used by an operator for an operation of controlling display of various types of information including an image on the display 30.

An outline of the present embodiment will be described.

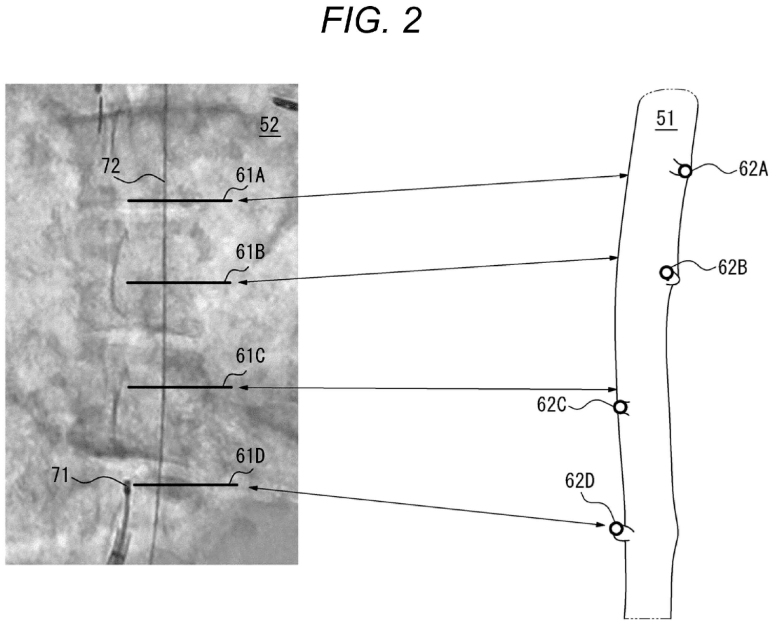

The image processing device 20 generates a three-dimensional image 51 as illustrated in Fig. 2. The three-dimensional image 51 is obtained by imaging a three-dimensional structure of a biological tissue based on tomographic information obtained by a sensor 71 that moves in a lumen of the biological tissue such as a blood vessel or a heart. The sensor 71 is, for example, an ultrasonic transducer used in IVUS. "IVUS" is an abbreviation for intravascular ultrasound. Upon receiving a calibration operation, the image processing device 20 stores the viewpoint information. The calibration operation is an operation of adjusting the viewpoint for displaying the three-dimensional image 51 on the display 30 in accordance with an emission direction of X-rays. The viewpoint information is information regarding the viewpoint adjusted by the calibration operation.

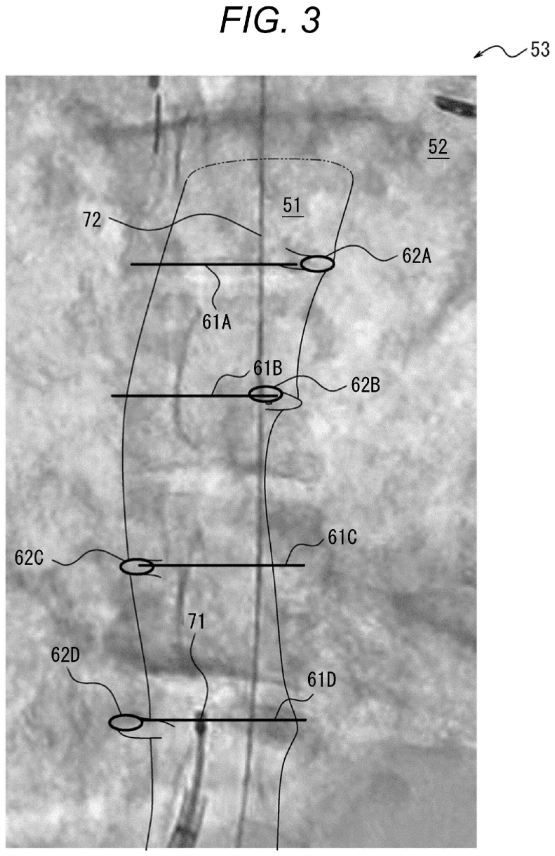

The image processing device 20 sequentially acquires X-ray images. Each X-ray image is a fluoroscopic view of a biological tissue using X-rays. Upon acquiring ratio information and position information, the image processing device 20 stores the acquired ratio information and position information. The ratio information is information regarding relative sizes of each X-ray image and the three-dimensional image 51 displayed on the display 30. The position information is information related to relative positions of each X-ray image and the three-dimensional image 51 displayed on the display 30. Each time the X-ray image is acquired, the image processing device 20 adjusts relative sizes and positions of the acquired X-ray image and the three-dimensional image 51 on the basis of the stored ratio information and position information, and then causes the display 30 to display the acquired X-ray image and three-dimensional image 51 so as to overlap each other as viewed from the viewpoint based on the stored viewpoint information. As a result, a superimposed image 53 as illustrated in Fig. 3 is displayed.

According to the present embodiment, the gap between the X-ray image and the three-dimensional image 51 can be reduced when the X-ray image and the three-dimensional image 51 are superimposed and displayed. For example, at the time of catheter surgery such as a stent graft indwelling procedure, it is conceivable to display the three-dimensional image 51 generated during surgery to be superimposed on the X-ray image generated in real time in order to reduce X-ray exposure or assist catheter operation. By superimposing the three-dimensional image 51 on the X-ray image, it is possible to simultaneously check the fine shape of the blood vessel and the real-time state. As illustrated in Figs. 2 and 3, when a hard guide wire 72 or catheter enters the blood vessel, the blood vessel is extended, but since the three-dimensional image 51 represents the extended blood vessel, a large gap does not occur between the three-dimensional image 51 and the X-ray image. In addition, it is possible to omit imaging of a three-dimensional CT image in which X-ray exposure may be a problem.

In the present embodiment, the image processing device 20 acquires at least one X-ray image 52 as illustrated in Fig. 2 in order to set the relative sizes and positions of each X-ray image and the three-dimensional image 51 displayed on the display 30. As the X-ray image 52, for example, it is conceivable to use an X-ray image acquired first among the X-ray images sequentially acquired by the image processing device 20. The image processing device 20 stores two or more positions in a first direction in the X-ray image 52 as first mark positions, and stores two or more positions in a direction corresponding to the first direction in the three-dimensional image 51 as second mark positions. In the example illustrated in Fig. 2, the image processing device 20 stores four positions in a longitudinal direction in the X-ray image 52 as first mark positions 61A, 61B, 61C, and 61D, and stores four positions in the longitudinal direction in the three-dimensional image 51 as second mark positions 62A, 62B, 62C, and 62D. The stored first mark positions and second mark positions are referred to when setting the relative sizes and positions of each X-ray image and the three-dimensional image 51 displayed on the display 30. Then, information regarding the set relative sizes and positions is stored as ratio information and position information, respectively.

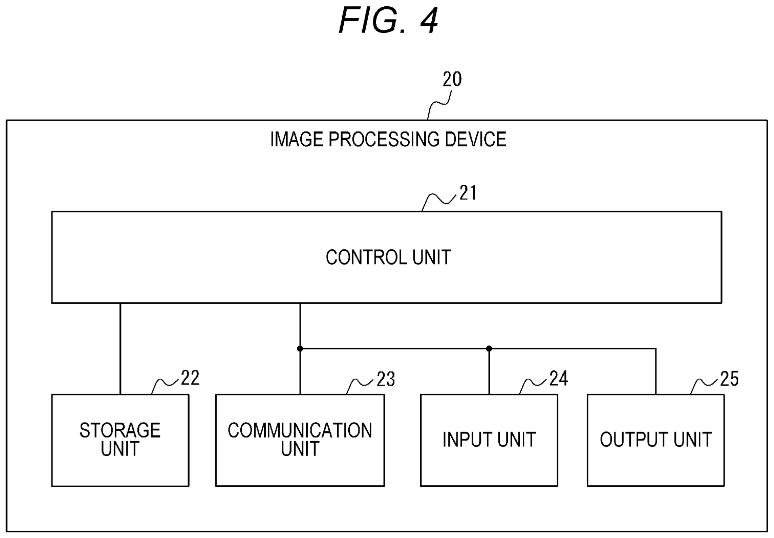

A configuration of the image processing device 20 according to the present embodiment will be described with reference to Fig. 4.

The image processing device 20 includes a control unit 21, a storage unit 22, a communication unit 23, an input unit 24, and an output unit 25.

The control unit 21 includes at least one processor, at least one programmable circuit, at least one dedicated circuit, or any combination thereof. The processor is a general-purpose processor such as a CPU or GPU, or a dedicated processor specialized for specific processing. "CPU" is an abbreviation for central processing unit. "GPU" is an abbreviation for graphics processing unit. The programmable circuit is, for example, an FPGA. "FPGA" is an abbreviation for field-programmable gate array. The dedicated circuit is, for example, an ASIC. "ASIC" is an abbreviation for application specific integrated circuit. The control unit 21 executes processing related to the operation of the image processing device 20 while controlling each unit of the image processing system 10 including the image processing device 20.

The storage unit 22 includes at least one semiconductor memory, at least one magnetic memory, at least one optical memory, or any combination thereof. The semiconductor memory is, for example, a RAM, a ROM, or a flash memory. "RAM" is an abbreviation for random access memory. "ROM" is an abbreviation for read only memory. The RAM is, for example, an SRAM or a DRAM. "SRAM" is an abbreviation for static random access memory. "DRAM" is an abbreviation for dynamic random access memory. The ROM is, for example, an EEPROM. "EEPROM" is an abbreviation for electrically erasable programmable read only memory. The flash memory is, for example, an SSD. "SSD" is an abbreviation for a solid-state drive. The magnetic memory is, for example, an HDD. "HDD" is an abbreviation for a hard disk drive. The storage unit 22 functions as, for example, a main storage device, an auxiliary storage device, or a cache memory. The storage unit 22 stores data to be used for the operation of the image processing device 20 and data obtained by the operation of the image processing device 20.

The communication unit 23 includes at least one communication module. The communication module is, for example, a module compatible with a wired LAN communication standard such as Ethernet (registered trademark) or a wireless LAN communication standard such as IEEE 802.11. "IEEE" is an abbreviation for Institute of Electrical and Electronics Engineers. The communication unit 23 receives data used for the operation of the image processing device 20 and transmits data obtained by the operation of the image processing device 20.

The input unit 24 includes at least one input interface. The input interface is, for example, a USB interface, an HDMI (registered trademark) interface, or an interface compatible with short-range wireless communication standards such as Bluetooth (registered trademark). "USB" is an abbreviation for universal serial bus. "HDMI (registered trademark)" is an abbreviation for High-Definition Multimedia Interface. The input unit 24 receives an operation of inputting data used for the operation of the image processing device 20. The input unit 24 is connected to the input device 40.

The output unit 25 includes at least one output interface. The output interface is, for example, a USB interface, an HDMI (registered trademark) interface, or an interface compatible with a short-range wireless communication standard such as Bluetooth (registered trademark). The output unit 25 outputs data obtained by the operation of the image processing device 20. The output unit 25 is connected to the display 30.

A function of the image processing device 20 is implemented by executing an image processing program according to the present embodiment by the processor corresponding to the control unit 21. That is, the function of the image processing device 20 is implemented by software. The image processing program causes a computer to function as the image processing device 20 by causing the computer to execute the operation of the image processing device 20. That is, the computer functions as the image processing device 20 by executing the operation of the image processing device 20 according to the image processing program.

The program can be stored in a non-transitory computer-readable medium. The non-transitory computer-readable medium is, for example, a flash memory, a magnetic recording device, an optical disc, a magneto-optical recording medium, or a ROM. Distribution of the program is executed by, for example, selling, transferring, or lending a portable medium such as an SD card, a DVD, or a CD-ROM storing the program. "SD" is an abbreviation for secure digital. "DVD" is an abbreviation for digital versatile disc. "CD-ROM" is an abbreviation for compact disc read only memory. The program may be distributed by being stored in a storage of a server in advance and transferred from the server to another computer. The program may be provided as a program product.

The computer temporarily stores, for example, the program stored in the portable medium or the program transferred from the server in the main storage device. Then, the computer reads, by the processor, the program stored in the main storage device, and executes, by the processor, processing according to the read program. The computer may read the program directly from the portable medium and execute the processing according to the program. Each time the program is transferred from the server to the computer, the computer may sequentially execute processing according to the received program. The processing may be executed by a so-called ASP-type service that implements a function only by an execution instruction and result acquisition without transferring the program from the server to the computer. "ASP" is an abbreviation for an application service provider. The programs include information that is used for processing by a computer and is equivalent to the programs. For example, data that is not a direct command to the computer but has a property that defines processing of the computer corresponds to the "information equivalent to the programs".

Some or all of the functions of the image processing device 20 may be implemented by a programmable circuit or a dedicated circuit as the control unit 21. That is, some or all of the functions of the image processing device 20 may be implemented by hardware.

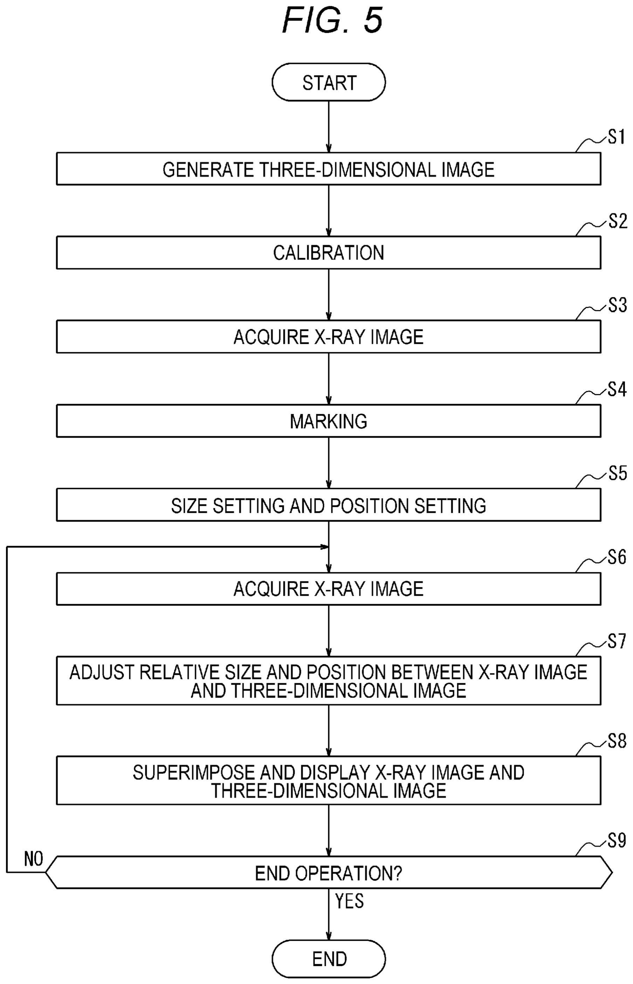

The operation of the image processing device 20 according to the present embodiment will be described with reference to Fig. 5. This operation corresponds to the image display method according to the present embodiment.

In step S1, the control unit 21 generates the three-dimensional image 51 as illustrated in Fig. 2. Specifically, the control unit 21 receives, via the communication unit 23, the tomographic information obtained by the sensor 71 that moves in a lumen of biological tissue such as a blood vessel or a heart. The tomographic information is, for example, reflected wave information regarding a reflected wave of an ultrasonic wave transmitted from the ultrasonic transducer as the sensor 71. The control unit 21 generates a plurality of cross-sectional images by imaging the cross-sectional structure of the biological tissue based on the received tomographic information. Alternatively, the control unit 21 may receive a plurality of cross-sectional images generated based on the tomographic information obtained by the sensor 71 from the IVUS device via the communication unit 23. The control unit 21 generates a three-dimensional image 51 by stacking a plurality of generated or received cross-sectional images. That is, the control unit 21 generates the three-dimensional image 51 by imaging the three-dimensional structure of the biological tissue based on the tomographic information obtained by the sensor 71.

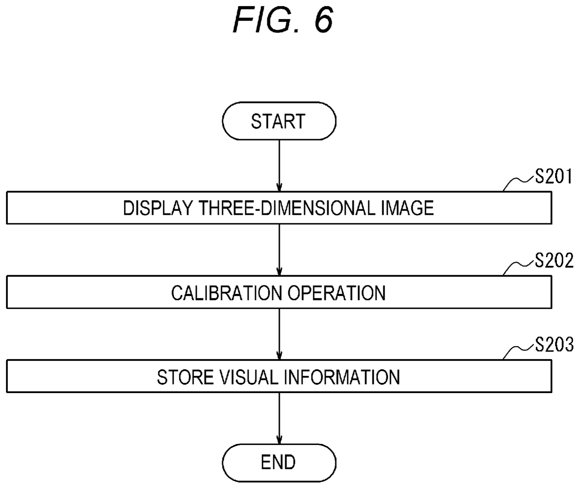

In step S2, the control unit 21 performs calibration. Fig. 6 illustrates a specific procedure of the processing in step S2.

In step S201, the control unit 21 causes the display 30 to display the three-dimensional image 51 generated in step S1. In step S202, the control unit 21 receives a calibration operation via the input device 40. The calibration operation is an operation of adjusting the viewpoint when the three-dimensional image 51 is displayed on the display 30 in accordance with the emission direction of X-rays from an X-ray device. For example, in step S1, it is assumed that the correspondence relationship between the orientation of a patient and the orientation of a cross-sectional image generated on the basis of the tomographic information is specified by a method using a marker attached to the catheter as disclosed in Patent Literature 1. Then, it is assumed that the sensor 71 is moved back and forth to generate a plurality of cross-sectional images, and the three-dimensional image 51 is further generated. In step S3 and subsequent steps, it is assumed that X-rays are emitted toward the front of the patient, that is, from the 12:00 direction using the X-ray device. In such a case, in the calibration operation, the viewpoint is adjusted so that a surface of the three-dimensional image 51 corresponding to the front of the patient is displayed on the display 30, that is, the three-dimensional structure of the biological tissue viewed from the 12:00 direction is displayed on the display 30 as the three-dimensional image 51. In step S203, the control unit 21 stores information regarding the viewpoint adjusted by the calibration operation in the storage unit 22 as viewpoint information.

In step S3, the control unit 21 acquires the X-ray image 52 as illustrated in Fig. 2. Specifically, the control unit 21 receives, as the X-ray image 52, an image obtained by fluoroscopic viewing a biological tissue with X-rays from the X-ray device through the communication unit 23.

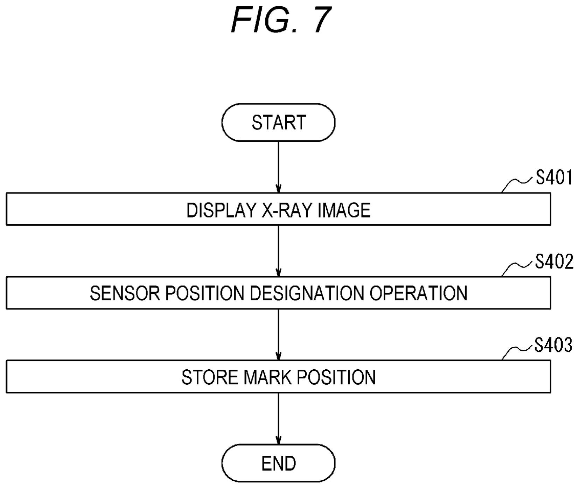

In step S4, the control unit 21 performs marking. Specifically, the control unit 21 stores two or more positions in the first direction in the X-ray image 52 acquired in step S3 in the storage unit 22 as first mark positions, and stores two or more positions in the direction corresponding to the first direction in the three-dimensional image 51 generated in step S1 in the storage unit 22 as second mark positions. Fig. 7 illustrates a specific procedure of the processing in step S4.

In step S401, the control unit 21 causes the display 30 to display the X-ray image 52 acquired in step S3. In step S402, the control unit 21 receives a sensor position designation operation via the input device 40. The sensor position designation operation is an operation of designating the position of the sensor 71 changed with the movement of the sensor 71 in the X-ray image 52 twice or more. In step S403, the control unit 21 stores the positions designated in step S402 in the storage unit 22 as first mark positions, and stores the positions corresponding to the positions of the sensor 71 when the sensor position designation operation is received in the three-dimensional image 51 generated in step S1 in the storage unit 22 as second mark positions.

For example, in the sensor position designation operation, the position of the destination of the sensor 71 in the X-ray image 52 is selected every time the sensor 71 is moved backward by a pull-back operation after the position of the sensor 71 in the X-ray image 52 is selected once. Assuming that the positions are selected four times, as in the example illustrated in Fig. 2, the four selected positions in the X-ray image 52 are stored in the storage unit 22 as the first mark positions 61A, 61B, 61C, and 61D. Then, the positions corresponding to the respective positions of the sensor 71 when the four positions are selected in the three-dimensional image 51 are stored in the storage unit 22 as the second mark positions 62A, 62B, 62C, and 62D.

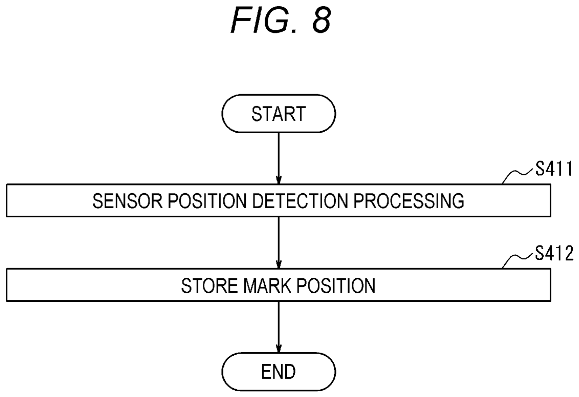

The position of the sensor 71 in the X-ray image 52 may be automatically sensed instead of being manually specified. Such a modification is illustrated in Fig. 8.

In step S411, the control unit 21 performs sensor position detection processing. The sensor position detection processing is processing of detecting the position of the sensor 71 changed with the movement of the sensor 71 in the X-ray image 52 twice or more. As a method of detecting the position of the sensor 71 in the X-ray image 52, a known image recognition technique can be used. Machine learning such as deep learning may be used. In step S412, the control unit 21 stores the positions detected in step S411 in the storage unit 22 as first mark positions, and stores the positions corresponding to the positions of the sensor 71 when the sensor position detection processing is performed in the three-dimensional image 51 generated in step S1 in the storage unit 22 as second mark positions.

For example, in sensor position designation processing, the position of the destination of the sensor 71 in the X-ray image 52 is detected every time the sensor 71 is moved backward by a pull-back operation after the position of the sensor 71 in the X-ray image 52 is detected once. Assuming that the positions are detected four times, as in the example illustrated in Fig. 2, the four detected positions in the X-ray image 52 are stored in the storage unit 22 as the first mark positions 61A, 61B, 61C, and 61D. Then, the positions corresponding to the respective positions of the sensor 71 when the four positions are detected in the three-dimensional image 51 are stored in the storage unit 22 as the second mark positions 62A, 62B, 62C, and 62D.

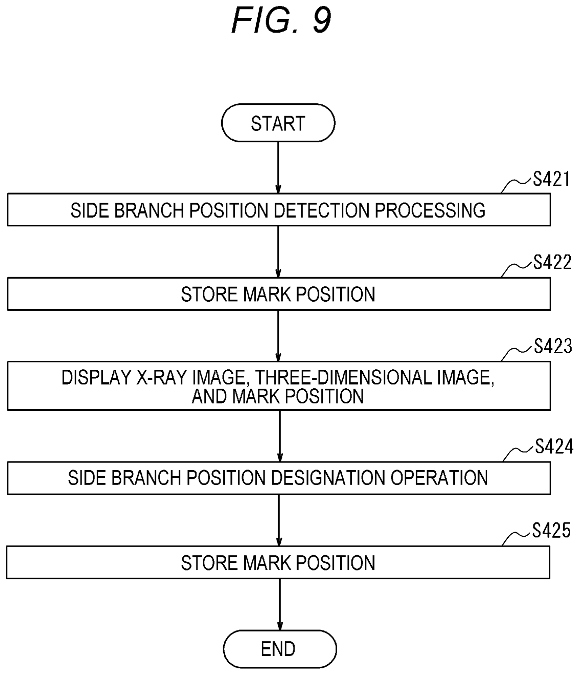

In a case where the biological tissue is branched at two or more positions separated in a major axis direction in the lumen to form a side branch, marking may be performed based on the position of the side branch in the X-ray image 52 instead of marking based on the position of the sensor 71 in the X-ray image 52. Such a modification is illustrated in Fig. 9.

In step S421, the control unit 21 performs side branch position detection processing. The side branch position detection processing is processing of detecting two or more positions of side branches in the three-dimensional image 51. As a method of detecting the position of the side branch in the three-dimensional image 51, a known image recognition technique can be used. Machine learning such as deep learning may be used. In step S422, the control unit 21 stores the positions detected in step S421 in the storage unit 22 as second mark positions. In step S423, the control unit 21 causes the display 30 to display the three-dimensional image 51, the second mark positions stored in the storage unit 22, and the X-ray image 52 acquired in step S3. In step S424, the control unit 21 receives a side branch position designation operation via the input device 40. The side branch position designation operation is an operation of designating two or more positions of the side branches corresponding to the second mark positions in the X-ray image 52. In step S425, the control unit 21 stores the positions designated in step S424 in the storage unit 22 as first mark positions.

For example, in a case where four positions at which the blood vessel branches in the three-dimensional image 51 are detected in the side branch position detection processing, as in the example illustrated in Fig. 2, the four detected positions in the three-dimensional image 51 are stored in the storage unit 22 as the second mark positions 62A, 62B, 62C, and 62D. Assuming that four branched positions of the blood vessel corresponding to the second mark positions 62A, 62B, 62C, and 62D in the X-ray image 52 are selected while confirming the second mark positions 62A, 62B, 62C, and 62D by the side branch position designation operation, the selected four positions in the X-ray image 52 are stored in the storage unit 22 as the first mark positions 61A, 61B, 61C, and 61D as in the example illustrated in Fig. 2.

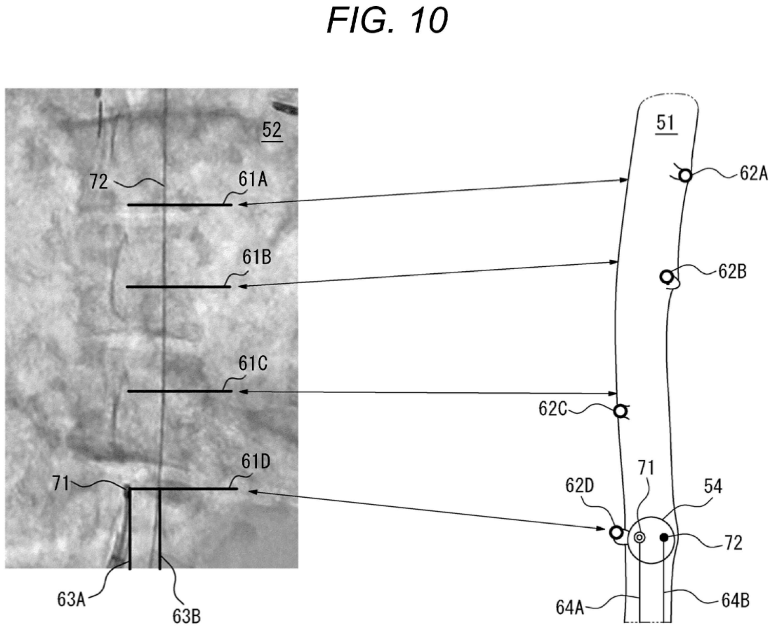

In step S4, the control unit 21 may further store two or more positions in a second direction orthogonal to the first direction in the X-ray image 52 acquired in step S3 in the storage unit 22 as third mark positions, and store two or more positions in a direction corresponding to the second direction in the three-dimensional image 51 generated in step S1 in the storage unit 22 as fourth mark positions. Similarly to the first mark positions and the second mark positions, the third mark positions and the fourth mark positions may be manually designated or may be automatically detected. In the example illustrated in Fig. 10, two positions in a lateral direction in the X-ray image 52 are stored in the storage unit 22 as the third mark positions 63A and 63B, and two positions in the lateral direction in the three-dimensional image 51 are stored in the storage unit 22 as the fourth mark positions 64A and 64B. More specifically, the current positions of the sensor 71 and the guide wire 72 in the lateral direction in the X-ray image 52 are stored in the storage unit 22 as the third mark positions 63A and 63B, and the current positions of the sensor 71 and the guide wire 72 in the lateral direction in a two-dimensional image 54 superimposed on the three-dimensional image 51 are stored in the storage unit 22 as the fourth mark positions 64A and 64B. The two-dimensional image 54 is obtained by imaging the cross-sectional structure of the biological tissue based on the tomographic information obtained by the sensor 71 at the current position of the sensor 71.

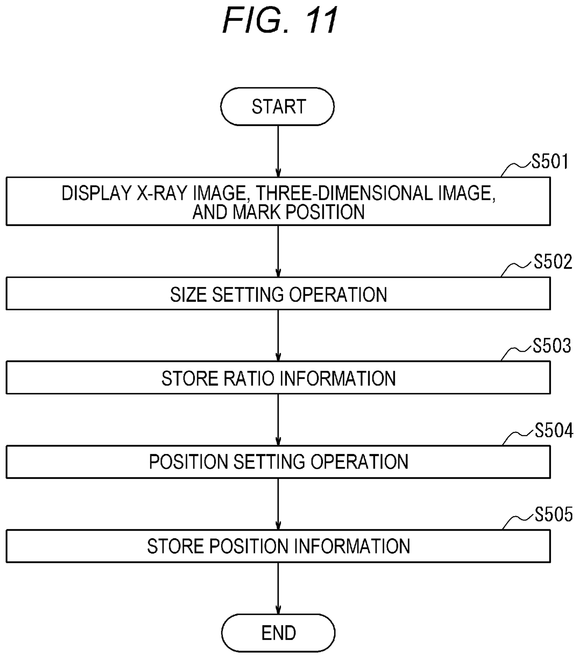

In step S5, the control unit 21 performs size setting and position setting with reference to the first mark positions and the second mark positions stored in the storage unit 22. Fig. 11 illustrates a specific procedure of the processing in step S5.

In step S501, the control unit 21 causes the display 30 to display the first mark positions and the second mark positions stored in the storage unit 22 together with the X-ray image 52 acquired in step S3 and the three-dimensional image 51 generated in step S1. In step S502, the control unit 21 receives a size setting operation via the input device 40. The size setting operation is an operation of setting the relative sizes of each X-ray image and the three-dimensional image 51 displayed on the display 30 by adjusting the relative sizes of the X-ray image 52 and the three-dimensional image 51 so that the distance between two first positions included in the first mark positions coincides with the distance between two second positions corresponding to the two first positions included in the second mark positions. In the example illustrated in Fig. 2, any two of the first mark positions 61A, 61B, 61C, and 61D can be the first positions. Of the second mark positions 62A, 62B, 62C, and 62D, two corresponding to the first positions are the second positions. In step S503, the control unit 21 acquires information regarding the relative sizes set by the size setting operation in step S502 as the ratio information, and stores the acquired ratio information in the storage unit 22. In step S504, the control unit 21 receives a position setting operation via the input device 40. The position setting operation is an operation of setting the relative positions of each X-ray image and the three-dimensional image 51 displayed on the display 30 by adjusting the relative positions of the X-ray image 52 and the three-dimensional image 51 so that the position of the sensor 71 in the X-ray image 52 overlaps with the position corresponding to the position of the sensor 71 in the three-dimensional image 51. Alternatively, the position setting operation may be an operation of setting the relative positions of each X-ray image and the three-dimensional image 51 displayed on the display 30 by adjusting the relative positions of the X-ray image 52 and the three-dimensional image 51 so that the position of the medical instrument such as the guide wire 72 inserted into the biological tissue separately from the sensor 71 in the X-ray image 52 overlaps with the position corresponding to the position of the medical instrument in the three-dimensional image 51. As in the example illustrated in Fig. 10, the two-dimensional image 54 may be superimposed on the three-dimensional image 51 and displayed so that the position of the sensor 71, the position of the guide wire 72, or both of them in the three-dimensional image 51 can be easily recognized. In step S505, the control unit 21 acquires information regarding the relative positions set by the position setting operation in step S504 as the position information, and stores the acquired position information in the storage unit 22.

In a case where the third mark positions and the fourth mark positions are further stored in the storage unit 22, the control unit 21 performs position setting with reference to the third mark positions and the fourth mark positions. Specifically, the control unit 21 receives the position setting operation in a state where the third mark positions and the fourth mark positions stored in the storage unit 22 are displayed on the display 30 together with the X-ray image 52 acquired in step S3 and the three-dimensional image 51 generated in step S1. The control unit 21 may receive the position setting operation in a state where the two-dimensional image 54 is superimposed on a predetermined position of the three-dimensional image 51 and displayed on the display 30.

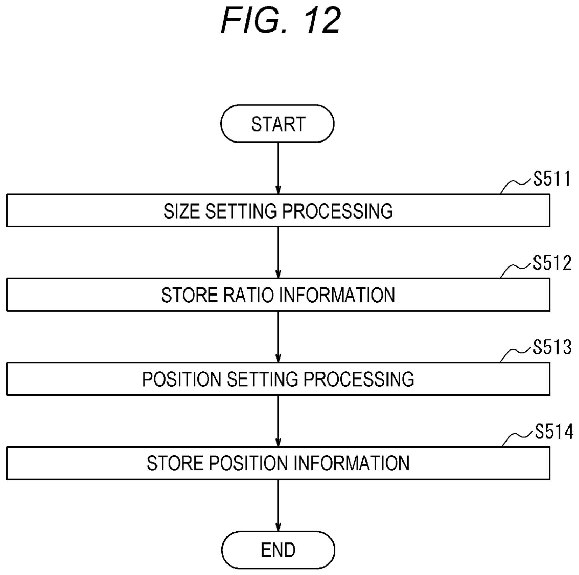

The relative sizes, positions, or both of them of each X-ray image and the three-dimensional image 51 displayed on the display 30 may be automatically set instead of being manually set. Such a modification is illustrated in Fig. 12.

In step S511, the control unit 21 performs size setting processing. The size setting processing is processing of setting the relative sizes of each X-ray image and the three-dimensional image 51 displayed on the display 30 by adjusting the relative sizes of the X-ray image 52 and the three-dimensional image 51 so that the distance between two first positions included in the first mark positions stored in the storage unit 22 coincides with the distance between two second positions corresponding to the two first positions included in the second mark positions stored in the storage unit 22. In the example illustrated in Fig. 2, any two of the first mark positions 61A, 61B, 61C, and 61D can be the first positions. Of the second mark positions 62A, 62B, 62C, and 62D, two corresponding to the first positions are the second positions. In step S512, the control unit 21 acquires information regarding the relative sizes set by the size setting processing in step S511 as ratio information, and stores the acquired ratio information in the storage unit 22. In step S513, the control unit 21 performs position setting processing. The position setting processing is processing of setting the relative positions of each X-ray image and the three-dimensional image 51 displayed on the display 30 by adjusting the relative positions of the X-ray image 52 and the three-dimensional image 51 so that the position of the sensor 71 in the X-ray image 52 overlaps with the position corresponding to the position of the sensor 71 in the three-dimensional image 51. Alternatively, the position setting processing may be processing of setting the relative positions of each X-ray image and the three-dimensional image 51 displayed on the display 30 by adjusting the relative positions of the X-ray image 52 and the three-dimensional image 51 so that the position of the medical instrument such as the guide wire 72 inserted into the biological tissue separately from the sensor 71 in the X-ray image 52 overlaps with the position corresponding to the position of the medical instrument in the three-dimensional image 51. For example, the position of the sensor 71, the position of the guide wire 72, or both of them in the three-dimensional image 51 may be identified with reference to the two-dimensional image 54 as illustrated in Fig. 10. In step S514, the control unit 21 acquires information regarding the relative positions set by the position setting processing in step S513 as the position information, and stores the acquired position information in the storage unit 22.

In step S6, the control unit 21 acquires the X-ray image. Specifically, similarly to step S3, the control unit 21 receives the X-ray image from the X-ray device through the communication unit 23.

In step S7, the control unit 21 adjusts the relative sizes and positions of the X-ray image acquired in step S6 and the three-dimensional image 51 generated in step S1 on the basis of the ratio information and the position information stored in the storage unit 22.

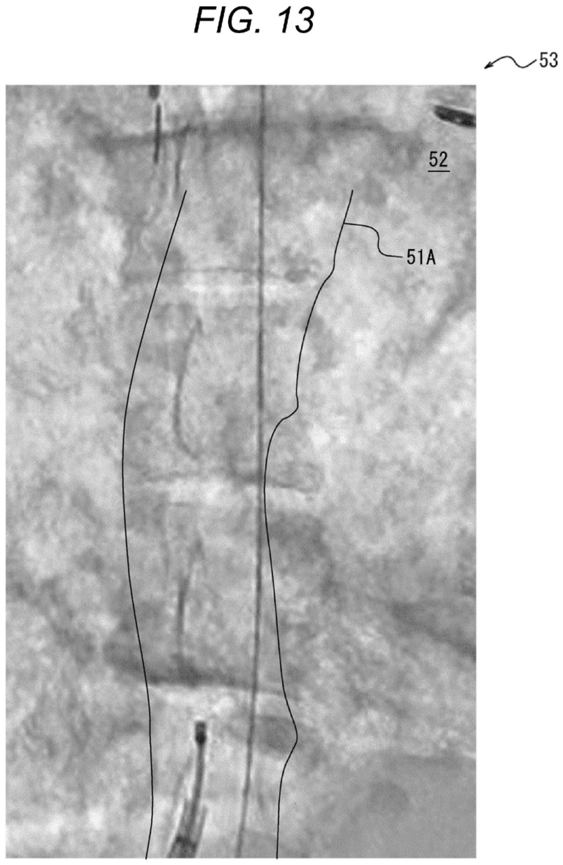

In step S8, the control unit 21 causes the display 30 to display the X-ray image and the three-dimensional image 51 having the relative sizes and positions adjusted in step S7 so as to overlap each other as viewed from the viewpoint based on the viewpoint information stored in the storage unit 22. As a result, the superimposed image 53 as illustrated in Fig. 3 is displayed. Specifically, the control unit 21 causes the display 30 to display the X-ray image and the three-dimensional image 51 so as to overlap each other as viewed from the viewpoint adjusted by the calibration operation in step S2 by arranging the X-ray image on the back side of the three-dimensional image 51 while making the three-dimensional image 51 translucent. Alternatively, the control unit 21 may cause the display 30 to display the X-ray image and the three-dimensional image 51 so as to overlap each other as viewed from the viewpoint adjusted by the calibration operation in step S2 by arranging the X-ray image in front of the three-dimensional image 51 while making the X-ray image translucent. As illustrated in Fig. 13, the superimposed image 53 may include only contour 51A for three-dimensional image 51. That is, when displaying the X-ray image and the three-dimensional image 51 on the display 30 so as to overlap each other as viewed from the viewpoint adjusted by the calibration operation in step S2, the control unit 21 may cause the display 30 to display only a contour of the three-dimensional image 51. The control unit 21 may capture angle information about the emission direction of the X-ray from the X-ray device and rotate the three-dimensional image 51 in accordance with the angle information.

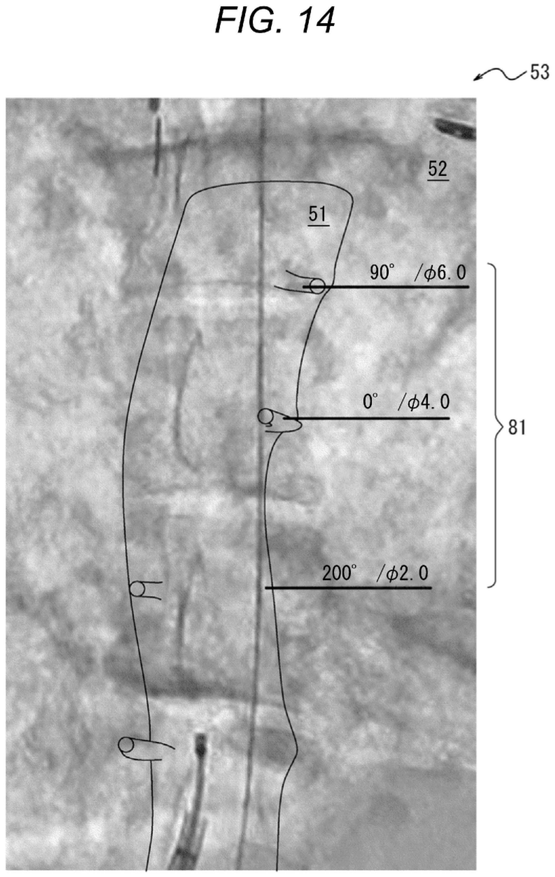

As illustrated in Fig. 14, the superimposed image 53 may be displayed together with side branch information 81. That is, when displaying the X-ray image and the three-dimensional image 51 on the display 30 so as to overlap each other as viewed from the viewpoint adjusted by the calibration operation in step S2, the control unit 21 may further cause the display 30 to display the side branch information 81. The side branch information 81 is information regarding a side branch. In the example illustrated in Fig. 14, the side branch information 81 includes information about the diameter of the lumen of the side branch and what angle the side branch extends at with respect to the currently visible image. For example, "90°/Φ6.0" indicates that the side branch vessel having a diameter of 6.0 mm extends in the right direction at a right angle with respect to the viewpoint. "0°/Φ4.0" indicates that the side branch vessel having a diameter of 4.0 mm extends toward the viewpoint. "200°/Φ2.0" indicates that the side branch vessel having a diameter of 2.0 mm extends in the left direction with respect to the viewpoint and slightly toward the viewpoint.

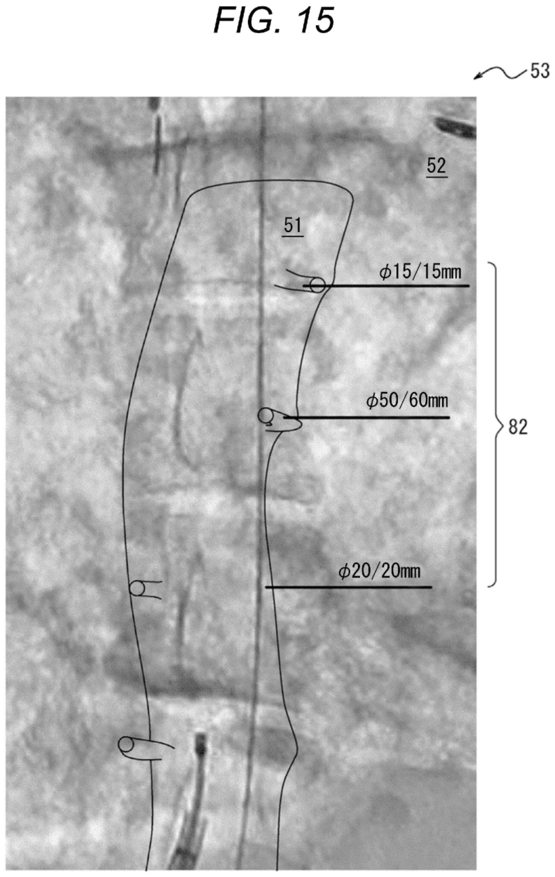

The superimposed image 53 may be displayed together with tube diameter information 82 as illustrated in Fig. 15. That is, when displaying the X-ray image and the three-dimensional image 51 on the display 30 so as to overlap each other as viewed from the viewpoint adjusted by the calibration operation in step S2, the control unit 21 may further cause the display 30 to display the tube diameter information 82. The tube diameter information 82 is information regarding the tube diameter of the lumen at the second mark position. In the example illustrated in Fig. 15, the tube diameter information 82 includes a tube diameter in a minor axis direction of the lumen and a tube diameter in a major axis direction of the lumen. For example, the minor axis and the major axis of a cross section of the lumen can be calculated by specifying the region of the lumen of the blood vessel from the two-dimensional image 54 using AI. "AI" is an abbreviation for artificial intelligence. "Φ50/60 mm" indicates that the minor axis of the cross-section of the lumen is 50 mm and the major axis is 60 mm. The aneurysm size, that is, the cross-sectional area of the lumen can also be calculated from the minor axis and the major axis of the cross-section of the lumen. Therefore, the cross-sectional area of the lumen may be displayed as the tube diameter information 82 instead of or in addition to the minor axis and the major axis of the cross-section of the lumen.

In step S9, upon receiving an end operation via the input device 40, the control unit 21 ends the operation illustrated in Fig. 5. If the end operation is not performed in step S9, the processing in and after step S6 is executed again.

In the present embodiment, every time the X-ray image is acquired in step S6, the processing in steps S7 and S8 is executed, so that the X-ray image can be continuously superimposed and displayed on the three-dimensional image 51 in the initially set direction, size, and position. Therefore, according to the present embodiment, it is possible to provide information useful for catheter operation while reducing X-ray exposure.

The present disclosure is not limited to the above-described embodiment. For example, two or more blocks illustrated in the block diagram may be integrated, or one block may be divided. Instead of executing two or more steps described in the flowchart in time series according to the description, the steps may be executed in parallel or in a different order according to the processing capability of the device that executes each step or as necessary. In addition, modifications can be made without departing from the gist of the present disclosure.

### Reference Signs List

10 Image processing system
20 Image processing device
21 Control unit
22 Storage unit
23 Communication unit
24 Input unit
25 Output unit
30 Display
40 Input device
51 Three-dimensional image
51A Contour
52 X-ray image
53 Superimposed image
54 Two-dimensional image
61A, 61B, 61C, 61D First mark position
62A, 62B, 62C, 62D Second mark position
63A, 63B Third mark position
64A, 64B Fourth mark position
71 Sensor
72 Guide wire
81 Side branch information
82 Tube diameter information

## Claims

1. An image processing device comprising:
a control unit that generates a three-dimensional image obtained by imaging a three-dimensional structure of a biological tissue based on tomographic information obtained by a sensor that moves in a lumen of the biological tissue, sequentially acquires X-ray images obtained by fluoroscopically viewing the biological tissue using an X-ray, receives a calibration operation of adjusting a viewpoint when the three-dimensional image is displayed on a display in accordance with a radiation direction of the X-ray, and acquires ratio information regarding relative sizes of each of X-ray images and the three-dimensional image displayed on the display, and position information regarding relative positions of each of the X-ray images and the three-dimensional image displayed on the display; and
a storage unit that stores viewpoint information regarding a viewpoint adjusted by the calibration operation, and the ratio information and the position information acquired by the control unit, wherein
each time an X-ray image is acquired, the control unit adjusts relative sizes and positions of the acquired X-ray image and the three-dimensional image on a basis of the ratio information and the position information stored in the storage unit, and then causes the display to display the acquired X-ray image and the three-dimensional image so as to overlap each other as viewed from a viewpoint based on the viewpoint information stored in the storage unit.

2. The image processing device according to claim 1, wherein
the control unit acquires at least one X-ray image, stores two or more positions in a first direction in the at least one X-ray image in the storage unit as first mark positions, and stores two or more positions in a direction corresponding to the first direction in the three-dimensional image in the storage unit as second mark positions, and
the first mark positions and the second mark positions stored in the storage unit are referred to when setting relative sizes and positions of each of the X-ray images and the three-dimensional image displayed on the display.

3. The image processing device according to claim 2, wherein the control unit receives a size setting operation of setting relative sizes of each of the X-ray images and the three-dimensional image displayed on the display in a state where the first mark positions and the second mark positions stored in the storage unit are displayed on the display together with the at least one X-ray image and the three-dimensional image, and acquires information regarding the relative sizes set by the size setting operation as the ratio information.

4. The image processing device according to claim 2, wherein the control unit performs size setting processing of setting relative sizes of each of the X-ray images and the three-dimensional image displayed on the display by adjusting relative sizes of each of the at least one X-ray image and the three-dimensional image so that a distance between two first positions included in the first mark positions stored in the storage unit coincides with a distance between two second positions corresponding to the two first positions included in the second mark positions stored in the storage unit, and acquires information regarding the relative sizes set by the size setting processing as the ratio information.

5. The image processing device according to claim 2, wherein the control unit receives a position setting operation of setting relative positions of each of the X-ray images and the three-dimensional image displayed on the display in a state where the first mark positions and the second mark positions stored in the storage unit are displayed on the display together with the at least one X-ray image and the three-dimensional image, and acquires information regarding the relative positions set by the position setting operation as the position information.

6. The image processing device according to claim 5, wherein the control unit stores two or more positions in a second direction orthogonal to the first direction in the at least one X-ray image in the storage unit as third mark positions, stores two or more positions in a direction corresponding to the second direction in the three-dimensional image in the storage unit as fourth mark positions, and receives the position setting operation in a state where the third mark positions and the fourth mark positions stored in the storage unit are displayed on the display together with the at least one X-ray image and the three-dimensional image.

7. The image processing device according to claim 5, wherein the control unit receives the position setting operation in a state where a two-dimensional image obtained by imaging a cross-sectional structure of the biological tissue based on the tomographic information is superimposed on a predetermined position of the three-dimensional image and displayed on the display.

8. The image processing device according to claim 2, wherein the control unit performs position setting processing of setting relative positions of each of the X-ray images and the three-dimensional image displayed on the display by adjusting relative positions of the at least one X-ray image and the three-dimensional image so that a position of the sensor in the at least one X-ray image overlaps with a position corresponding to the position of the sensor in the three-dimensional image after relative sizes of the at least one X-ray image and the three-dimensional image is adjusted so that a distance between two first positions included in the first mark positions stored in the storage unit coincides with a distance between two second positions corresponding to the two first positions included in the second mark positions stored in the storage unit, and acquires information regarding the relative positions set by the position setting processing as the position information.

9. The image processing device according to claim 2, wherein the control unit performs position setting processing of setting relative positions of each of the X-ray images and the three-dimensional image displayed on the display by adjusting relative positions of the at least one X-ray image and the three-dimensional image so that a position of a medical instrument inserted into the biological tissue separately from the sensor in the at least one X-ray image overlaps with a position corresponding to the position of the medical instrument in the three-dimensional image after relative sizes of the at least one X-ray image and the three-dimensional image is adjusted so that a distance between two first positions included in the first mark positions stored in the storage unit coincides with a distance between two second positions corresponding to the two first positions included in the second mark positions stored in the storage unit, and acquires information regarding the relative positions set by the position setting processing as the position information.

10. The image processing device according to claim 9, wherein the medical instrument is a guide wire.

11. The image processing device according to claim 2, wherein the control unit receives a sensor position designation operation of designating a position of the sensor changed with movement of the sensor in the at least one X-ray image twice or more, stores designated positions in the storage unit as the first mark positions, and stores positions corresponding to the position of the sensor when the sensor position designation operation is received in the three-dimensional image in the storage unit as the second mark positions.

12. The image processing device according to claim 2, wherein the control unit performs sensor position detection processing of detecting a position of the sensor changed with movement of the sensor in the at least one X-ray image twice or more, stores detected positions in the storage unit as the first mark positions, and stores positions corresponding to the position of the sensor when the sensor position detection processing is performed in the three-dimensional image in the storage unit as the second mark positions.

13. The image processing device according to claim 2, wherein
the biological tissue is branched to form side branches at two or more locations separated in a major axis direction in the lumen, and
the control unit performs side branch position detection processing of detecting positions of the side branches in the three-dimensional image, and stores detected positions in the storage unit as the second mark positions.

14. The image processing device according to claim 13, wherein, when causing the display to display each of the X-ray images and the three-dimensional image so as to overlap each other as viewed from the viewpoint, the control unit further causes the display to display side branch information regarding the side branches.

15. The image processing device according to claim 2, wherein, when causing the display to display each of the X-ray images and the three-dimensional image so as to overlap each other as viewed from the viewpoint, the control unit further causes the display to display tube diameter information regarding a tube diameter of the lumen at the second mark positions.

16. The image processing device according to claim 1, wherein the control unit causes the display to display each of the X-ray images and the three-dimensional image so as to overlap each other as viewed from the viewpoint by arranging each of the X-ray images on a back side of the three-dimensional image while making the three-dimensional image translucent.

17. The image processing device according to claim 1, wherein the control unit causes the display to display each of the X-ray images and the three-dimensional image so as to overlap each other as viewed from the viewpoint by arranging each of the X-ray images in front of the three-dimensional image while making each of the X-ray images translucent.

18. The image processing device according to claim 1, wherein, when causing the display to display each of the X-ray images and the three-dimensional image so as to overlap each other as viewed from the viewpoint, the control unit causes the display to display only a contour of the three-dimensional image.

19. An image processing system comprising: the image processing device according to any one of claims 1 to 18; and the display.

20. An image display method comprising:
generating a three-dimensional image obtained by imaging a three-dimensional structure of a biological tissue based on tomographic information obtained by a sensor that moves in a lumen of the biological tissue;
sequentially acquiring X-ray images obtained by fluoroscopically viewing the biological tissue using an X-ray;
receiving a calibration operation of adjusting a viewpoint when the three-dimensional image is displayed on a display in accordance with a radiation direction of the X-ray; and
acquiring ratio information regarding relative sizes of each of X-ray images and the three-dimensional image displayed on the display, and position information regarding relative positions of each of the X-ray images and the three-dimensional image displayed on the display, wherein
each time an X-ray image is acquired, relative sizes and positions of the acquired X-ray image and the three-dimensional image are adjusted on a basis of the ratio information and the position information, and then the display is caused to display the acquired X-ray image and the three-dimensional image so as to overlap each other as viewed from the viewpoint adjusted by the calibration operation.

21. An image processing program for causing a computer to execute an operation comprising:
generating a three-dimensional image obtained by imaging a three-dimensional structure of a biological tissue based on tomographic information obtained by a sensor that moves in a lumen of the biological tissue;
sequentially acquiring X-ray images obtained by fluoroscopically viewing the biological tissue using an X-ray;
receiving a calibration operation of adjusting a viewpoint when the three-dimensional image is displayed on a display in accordance with a radiation direction of the X-ray; and
acquiring ratio information regarding relative sizes of each of X-ray images and the three-dimensional image displayed on the display, and position information regarding relative positions of each of the X-ray images and the three-dimensional image displayed on the display, wherein
each time an X-ray image is acquired, relative sizes and positions of the acquired X-ray image and the three-dimensional image are adjusted on a basis of the ratio information and the position information, and then the display is caused to display the acquired X-ray image and the three-dimensional image so as to overlap each other as viewed from the viewpoint adjusted by the calibration operation.
